# EUROPEAN PATENT SPECIFICATION

(11) **EP 4 337 783 B1**
(45) Date of publication and mention of the grant of the patent: **03.12.2025**
(21) Application number: 22727204.4
(22) Date of filing: 20.05.2022
(51) Int. Cl.: C12Q 1/00, D01D 1/02, D01D 5/00, D01F 9/17, G01N 27/327

(54) **IMPROVEMENTS RELATING TO ELECTROCHEMICAL SENSORS**
VERBESSERUNGEN IN BEZUG AUF ELEKTROCHEMISCHE SENSOREN
AMÉLIORATIONS SE RAPPORTANT À DES CAPTEURS ÉLECTROCHIMIQUES

(30) Priority: 21.05.2021 GB 202107313
(43) Date of publication of application: 20.03.2024
(73) Proprietor: University of Limerick, Limerick V94 T9PX (IE)
(72) Inventor: COLLINS, Maurice, Limerick Plassey Technological Park Limerick, V94 T9PX (IE); RUBIO, Mario, Culebras, Limerick Plassey Technological Park Limerick, V94 T9PX (IE); MCLOUGHLIN, Anne, Beaucamp, Limerick Plassey Technological Park Limerick, V94 T9PX (IE)
(74) Representative: Appleyard Lees IP LLP
(86) International application number: PCT/EP2022/063815
(87) International publication number: WO 2022/243555

(56) References cited:
- CN-B- 109 704 334
- TAE-SUNG BAE ET AL: "Effects of carbon structure orientation on the performance of glucose sensors fabricated from electrospun carbon fibers", JOURNAL OF NON-CRYSTALLINE SOLIDS, vol. 358, no. 3, 24 November 2011 (2011-11-24), pages 544 - 549, XP028446970, ISSN: 0022-3093, [retrieved on 20111109], DOI: 10.1016/J.JNONCRYSOL.2011.11.002
- WANG SHICHAO ET AL: "Lignin-based carbon fibers: Formation, modification and potential applications", GREEN ENERGY & ENVIRONMENT, 8 April 2021 (2021-04-08), pages 1 - 46, XP055879790, Retrieved from the Internet <URL:https://www.sciencedirect.com/science/article/pii/S2468025721000686> [retrieved on 20220117]
- BEAUCAMP ANNE ET AL: "Sustainable mesoporous carbon nanostructures derived from lignin for early detection of glucose", vol. 23, no. 15, 2 August 2021 (2021-08-02), GB, pages 5696 - 5705, XP055879088, ISSN: 1463-9262, Retrieved from the Internet <URL:https://pubs.rsc.org/en/content/articlepdf/2021/gc/d1gc02062e> DOI: 10.1039/D1GC02062E
- DEMIROGLU MUSTAFOV SIBEL ET AL: "Fabrication of conductive Lignin/PAN carbon nanofibers with enhanced graphene for the modified electrodes", CARBON, vol. 147, 25 February 2019 (2019-02-25), GB, pages 262 - 275, XP055879612, ISSN: 0008-6223, DOI: 10.1016/j.carbon.2019.02.058
- CULEBRAS MARIO ET AL: "Bio-derived Carbon Nanofibres from Lignin as High-Performance Li-Ion Anode Materials", vol. 12, no. 19, 12 September 2019 (2019-09-12), DE, pages 4516 - 4521, XP055879105, ISSN: 1864-5631, Retrieved from the Internet <URL:https://onlinelibrary.wiley.com/doi/full-xml/10.1002/cssc.201901562> DOI: 10.1002/cssc.201901562
- ELENI SAPOUNTZI ET AL: "Recent Advances in Electrospun Nanofiber Interfaces for Biosensing Devices", SENSORS, vol. 17, no. 8, 16 August 2017 (2017-08-16), pages 1887, XP055525650, DOI: 10.3390/s17081887

## Description

### Field

The present invention relates to a method of forming an electrode for a sensor device. The present invention also relates to an electrode, a sensor device comprising said electrode, a use of a mesoporous carbon nanofibre to immobilise an enzyme on an electrode and a method of detecting a biomarker using said electrodes and device. In particular, the present invention relates to a mesoporous carbon nanofibre produced from polylactic acid and lignin for immobilising enzymes useful in chemical or biomarker sensing applications.

### Background

Carbon nanomaterials have a diverse range of uses in industry and technology. One such use is in electrodes of highly sensitive chemical sensor devices, for example sensors for measuring the glucose level of a patient with diabetes. Patients with diabetes need to regularly measure their glucose level to effectively manage their condition. This is typically done by using "finger-prick" tests which involve the patient withdrawing a small amount of blood and analysing the blood with a test kit. Repeated "finger-prick" tests can be inconvenient and painful for patients. An alternative means for measuring glucose levels in a patient is provided by the glucose sensor disclosed in WO 2014/110492 A2 which is sensitive enough to allow the measurement of glucose in a saliva sample. This is much more convenient for a patient than testing a blood sample. The glucose sensor includes an insulating or semiconducting substrate, a working electrode, a counter electrode, and a reference electrode, as well as a sample placement area on the surface of the substrate for receiving the saliva sample. The working electrode comprises a conductive metal layer deposited on the substrate in the sample placement area. The working electrode also comprises single-walled carbon nanotubes and a coating containing glucose oxidase. The working electrode, counter electrode, and reference electrode are each connected to an amperometry circuit, whose output voltage provides a measure of the glucose concentration in the liquid sample deposited in the sample placement area. The output signal of the detection circuit is correlated to the glucose concentration in the liquid sample. The glucose sensor is said to be highly sensitive and able to detect glucose levels at 5 ppm.

Carbon nanofibers in particular are promising materials for various applications. Currently, the vast majority of the carbon nanofibres are produced by electrospinning of polyacrylonitrile (PAN), which is derived from petroleum sources, and by carbon vapor deposition. There are several disadvantages to the use of PAN as a carbon nanofibre precursor material. For example high cost, slow carbonization and certain environmental problems associated with PAN production. Therefore there is a need for a more sustainable source of carbon nanofibers.

Lignin may be considered as a green alternative for carbon fibre production. Lignin along with hemicellulose and cellulose is one of the most abundant components of lignocellulosic biomass and can be sustainably produced. Lignin is amorphous and the only aromatic biopolymer present in the cell walls of pith, roots, fruit, buds and bark. However, there are limitations to the use of lignin in electrospinning such as low viscosity and low quality of the fibres produced, which makes industrial scalability of carbon fibre production from lignin extremely complicated. It would therefore be beneficial to improve the electrospinning behaviour of lignin-derived precursor fibres.

Bae et al (T.-S. Bae et al. Effects of carbon structure orientation on the performance of glucose sensors fabricated from electrospun carbon fibers. Journal of Non-Crystalline Solids. 2012, Vol. 358, pages 544 to 549) discloses a glucose sensor comprising an electrode with a mesoporous carbon nanofibre layer made from polyacrylonitrile and silica as activating agent. The glucose oxidase enzyme immobilization was developed based on improved specific surface area and pore volume of the carbon nanofibre layer. Shichao et al. (Shichao et al. Lignin-based carbon fibers: Formation, modification and potential applications. Green Energy and Environment. 8 April 2021. Online) is a review on the formation of lignin fibers and nanofibers and their uses.

### Summary of the Invention

It is one aim of the present invention, amongst others, to provide an electrode for a sensor device that addresses at least one disadvantage of the prior art, whether identified here or elsewhere, or to provide an alternative to existing electrodes and sensor devices. For instance, it may be an aim of the present invention to provide an electrode for a sensor device formed from sustainable materials which can effectively immobilise an enzyme for detecting a target compound or biomarker. According to aspects of the present invention, there is provided an electrode for a sensor device, a method of forming said electrode, a sensor device comprising said electrode, a use of a mesoporous carbon nanofibre material for immobilising an enzyme in a sensor device and a method of detecting a target compound or biomarker using said electrode, as set forth in the appended claims. Other features of the invention will be apparent from the dependent claims, and from the description which follows.

According to a first aspect of the present invention, there is provided a method of forming an electrode for a sensor device, the method comprising the steps of:
a) forming fibres on a substrate, the fibres comprising lignin and a second polymeric material, wherein the second polymeric material is immiscible with lignin;
b) carbonising the fibres formed in step a) to provide a carbon nanofibre layer on the substrate; wherein the carbon nanofibre layer comprises mesoporous carbon nanofibers; and
c) immobilising an enzyme on the carbon nanofibre layer, in mesopores of the carbon nanofibres.

The method of this first aspect suitably provides an electrode for use in a sensor device which functions by detecting a change in output voltage of the device when a target molecule interacts with the enzyme immobilised on the carbon nanofibre layer. For example, the electrode may be used in a glucose sensor device wherein the enzyme immobilised on the carbon nanofiber layer is glucose oxidase (GOx). The inventors have found that carbon nanofibers formed from precursor fibres comprising lignin and the second polymeric material provide, after carbonization, carbon nanofibers which are particularly effective for the immobilisation of such enzymes in order to produce an electrode for enzyme-based sensor devices. This advantage is believed to be due to the porosity of the carbon nanofibers produced in this manner. The carbon nanofibres so produced provide a conductive carbon nanofibre framework comprising mesopores which have been found to be effective for immobilisation of enzymes such as glucose oxidase. The mesopores bind and retain the enzyme in the carbon nanofibre framework which exposes the immobilised enzymes over a relatively large surface area of the carbon nanofibres to a test sample applied to the electrode. The conductive properties of the carbon nanofibre network in effect connect the enzymes to the circuit of the sensor device so that electrochemical reactions catalysed by the enzymes can be detected by the sensor device. Therefore the carbon nanofibre material described herein has been found to be particularly effective for this application.

Also, using said composition comprising lignin and the second polymeric material to form the electrode allows the cost of production to be lowered due to the relatively low cost of these materials and also improves the environmental profile of such electrodes due to these materials being obtainable from sustainable sources, especially when the second polymeric material is obtained from a renewable source, for example wherein the second polymeric material polylactic acid.

Furthermore, the composition of the fibres can be varied in the amounts of lignin, the second polymeric material and any further components present in order to produce a carbon nanofiber layer with specific properties, such as pore size and/or distribution, for immobilising specific enzymes. Forming the carbon nanofibres from this variable composition allows this advantageous tuning of the properties of the carbon nanofibre layer.

Suitably the steps of the method are carried out in the order step a) followed by step b) followed by step c).

The method of this first aspect provides an electrode for a sensor device, more specifically an electrochemical sensor device. Suitably the electrode is adapted for use as a working electrode in such a sensor device, for example for use with a counter electrode and a reference electrode. Suitable arrangements of said electrodes in which the working electrode of the present invention can be used are known in the art.

Step a) of the method involves forming fibres on a substrate, the fibres comprising lignin. Step a) may involve forming the fibres by electrospinning, for example by electrospinning a solution of the lignin and the second polymeric material onto the substrate. The fibres formed in step a) may be referred to as precursor fibres or carbon nanofibre precursor fibres.

It is believed that any type of lignin can be utilised in the method of this first aspect, for example lignin obtained from softwood, hardwood or grass/annual plants. Suitable lignin can be obtained from these sources using various known processes, for example the Kraft, organosolve or soda processes. In some embodiments, more than one type and/or source of lignin is used to provide the lignin of the composition. An example of a suitable lignin for use in the method is Alcell Organosolv hardwood lignin (AL) obtained from Tecnaro GmbH.

The second polymeric material may be any suitable polymeric material which is immiscible with lignin. Suitably the second polymeric material has a low carbon yield on carbonisation, suitably below 10% or below 5%. The second polymeric material may be selected from polyethylene, polypropylene, polyamides, polyetherimide, polystyrene, polyacrylates (e.g. PMMA), polycarbonate, polyvinyl acetate, polyvinyl chloride, polylactic acid or blends/mixtures thereof. Suitably the second polymeric material is a bio-based polymeric material obtainable from an environmentally sustainable source. Suitably the second polymeric material is polylactic acid.

The process used for forming the fibres on the substrate can be adjusted to alter the composition of the fibres formed. For example, the relative amounts of the lignin and the second polymeric material in the solution used for electrospinning can be varied to produce fibres with different relative amounts of lignin and the second polymeric material. Varying the composition in this way can adjust the processing properties of the fibres and/or the structure and/or porosity of the carbon nanofibres produced by the method, which may be advantageous for adapting the electrode to different specific uses as discussed above.

Suitably the fibres comprise at least 30 wt% lignin, suitably at least 35 wt%, suitably at least 40 wt% or at least 45 wt% lignin.

Suitably the fibres comprise up to 90 wt% lignin, suitably up to 80 wt%, suitably up to 70 wt% or up to 60 wt% lignin.

Suitably the fibres comprise 30-70 wt% lignin, suitably 40-60 wt% lignin, suitably 40-50 wt% lignin.

Suitably the fibres comprise at least 10 wt% of the second polymeric material, suitably at least 20 wt%, suitably at least 30 wt% or at least 40 wt% of the second polymeric material, suitably polylactic acid.

Suitably the fibres comprise up to 70 wt% of the second polymeric material, suitably up to 60 wt%, suitably up to 55 wt% or up to 50 wt% of the second polymeric material, suitably polylactic acid.

Suitably the fibres comprise 30-70 wt% of the second polymeric material, suitably 40-60 wt%, suitably 45-55 wt% of the second polymeric material, suitably polylactic acid.

Suitably the fibres of step a) comprise 30-70 wt% of the second polymeric material and 30-70 wt% lignin, suitably 40-60 wt% of the second polymeric material and 40-60 wt% lignin, suitably wherein the second polymeric material is polylactic acid. In some embodiments, the fibres comprise 45-55 wt% of the second polymeric material and 40-50 wt% lignin, suitably wherein the second polymeric material is polylactic acid.

Suitably the fibres of step a) comprise a crosslinking agent. The crosslinking agent is suitably a reactive species which forms crosslinks between polymer chains of lignin. The crosslinking agent may be selected from diisocyanates such as toluene diisocyanate or methylene diphenyl diisocyanate, or from diglycidyl ethers such as neopentyl glycol diglycidyl ether, bisphenol A diglycidyl ether and 1,4-butanediol diglycidyl ether. In some embodiments, the crosslinking agent is methylene diphenyl diisocyanate (MDI).

Suitably the fibres of step a) comprise at least 1 wt% of the crosslinking agent, suitably at least 2 wt% or at least 3 wt%.

Suitably the fibres of step a) comprise up to 10 wt% of the crosslinking agent, suitably up to 8 wt% or up to 5 wt%.

Suitably the fibres of step a) comprise 1-10 wt% of the crosslinking agent, suitably 2-8 wt%, suitably 2-5 wt% or 3-4 wt% of the crosslinking agent, suitably of MDI.

In view of the details of the present invention provided herein, the skilled person would be able to adapt the process of forming the fibres in order to provide fibres comprising the above amounts of lignin and the second polymeric material.

Suitably the substrate on which the fibres are formed in step a) comprises an insulating layer (i.e. an electrically insulating layer). The insulating layer may be provided by any suitable electrically insulating material, preferably a chemically inert electrically insulating material, suitably selected from silicon, glass, ceramic, quartz, a non-conductive polymer, and combinations thereof. Suitably the insulating material is formed from quartz.

Suitably the substrate comprises a conductive layer (i.e. an electrically conductive layer). The conductive layer may be provided by any suitable electrically conductive material, suitably a chemically inert electrically conductive material, suitably selected from the group consisting of gold, platinum, iridium, silver, silver/silver chloride, and combinations thereof. Suitably the conductive layer is formed from gold.

Suitably the substrate comprises an insulating layer and a conductive layer. Suitably the conductive layer is bonded to the insulating layer. In some embodiments, the conductive layer is a layer of gold provided on the insulating layer, for example a layer of gold on a layer of quartz.

Suitably the fibres are formed on the conductive layer.

Suitably the method of this first aspect comprises a step a2) of stabilising the fibres by exposing the fibres on the substrate to a temperature of at least 100°C, suitably at least 150°C or at least 200°C, suitably in air. Suitably step a2) involves exposing the fibres on the substrate to a temperature of up to 300°C, suitably up to 250°C, suitably in air. Step a2) suitably involves heating the fibres to a target temperature of from 100°C to 300°C or from 150°C to 250°C, suitably gradually over at least one hour, suitably over at least two hours or at least three hours. Step a2) suitably involves exposing the fibres to the target temperature, once that temperature has been reached, for a period of at least one hour, suitably at least two hours. Step a2) may involve halting the heating of the fibres at at least one intermediate temperature, suitably at more than one intermediate temperature, for at least one hour or for at least two hours, before increasing the temperature to the next intermediate temperature or to the target temperature. Step a2) suitably converts the thermoplastic lignin-based fibres into thermoset crosslinked polymer fibres. Performing step a2) may prevent melting of the lignin-based fibres during the carbonisation step b).

The method of this first aspect comprises step b) of carbonising the fibres formed in step a) to provide a carbon nanofibre layer on the substrate. Suitably step b) involves exposing the fibres on the substrate to a temperature of at least 700°C, suitably to a temperature of at least 800°C, for example approximately 900°C. Suitably step b) involves heating the fibres to such temperatures gradually over at least one hour. Suitably step b) involves heating the fibres at such temperatures for sufficient time to affect carbonisation of the fibres, for example at least 10 minutes, at least 20 minutes or at least 30 minutes. Step b) may be carried out in a tubular furnace.

After step b) the fibres are substantially carbonised wherein the lignin has turned to carbon and the second polymeric material (such as polylactic acid) has substantially evaporated from the precursor fibres to provide the carbon nanofibres of the carbon nanofibre layer.

The carbon nanofibres of the carbon nanofibre layer are mesoporous. The phase separation and pyrolysis of the second polymeric material from the crosslinked network of lignin during step b) suitably produces said mesopores. The inventors have found that the mesopores having a size in the range 2-4 nm which are formed in the carbon nanofibres in step b) are particularly advantageous for the immobilisation of enzymes such as glucose oxidase. Suitably the carbon nanofibres have a relative mesopores volume ratio of at least 30%, suitably at least 40% or at least 50%. This relative mesopores volume ratio can be obtained from standard measurements using Brunauer-Emmett-Teller (BET) analysis.

Suitably the carbon nanofibers produced in step b) are porous or mesoporous with a BET surface area of at least 500 m²g⁻¹, suitably in the range 500 to 2,000 m²g⁻¹. Known methods of standard BET analysis can provide this BET surface area.

The carbon nanofibre layer is suitably sufficiently conductive to allow electron transfer between an enzyme immobilised in the carbon nanofibre layer and the conductive layer of the substrate.

Step c) of the method of this first aspect involves immobilising an enzyme on the carbon nanofibre layer. Any suitable enzyme which can associate with the mesopores of the carbon nanofibres to retain the enzyme in the carbon nanofibre layer and which can produce a detectable electrochemical change on binding to a target compound or biomarker, may be used. Suitably the enzyme immobilised in the carbon nanofibre layer is capable of affecting the output voltage of the electrode in the presence or absence of the target compound or biomarker. Suitably the enzyme is capable of proportionally affecting the voltage output of the electrode according to the concentration of the target compound or biomarker contacting the carbon nanofibre layer, for example in a liquid sample deposited on the electrode.

Suitably step c) involves applying a solution of the enzyme to the carbon nanofibre layer. The loading of the enzyme on to the carbon nanofibre layer can be varied according to the specific requirements of the sensor device. For example, a solution of enzyme having a concentration of from 2 to 50 mg.ml⁻¹, for example around 5 mg.ml⁻¹, suitably in a phosphate buffer solution (PBS).

Suitably the electrode is dried after applying the solution of the enzyme to the carbon nanofibre layer to remove the solvent of the solution and leave the enzyme immobilised on the carbon nanofibre layer. The electrode may be dried at a temperature of from 20 to 50°C, suitably from 30 to 40°C. The electrode may be dried at such temperatures for at least one hour, suitably for at least 2 hours or at least three hours.

Suitably step c) provides a loading of enzyme on the carbon nanofibre layer sufficient to provide a monolayer of the enzyme on the carbon nanofibre layer.

The enzyme immobilised on the carbon nanofibre layer is suitably adsorbed to the mesopores of the carbon nanofibers and held in place by weak intermolecular forces.

The method of this first aspect may comprise, after step c), a step d) of applying a protective coating to the carbon nanofibre layer. Suitably the protective coating is a permeable or semi-permeable membrane. The protective coating may comprise fluorocarbon polymeric material, for example Nafion^{™}. The protective coating may be formed by applying Nafion to the carbon nanofibre layer comprising the enzyme. Suitably the protective coating allows a sample solution to permeate through to the carbon nanofibre layer in use.

In some embodiments, the enzyme of step c) is glucose oxidase and the sensor device is a glucose sensor which can measure the concentration of glucose in a liquid sample, for example a saliva sample from a patient, when the sample is deposited on the carbon nanofibre layer of the electrode, due to the change in output voltage of the electrode produced by the reaction of the glucose oxidase enzyme with glucose in the sample.

The method of this first aspect may comprise a further step e) of incorporating the electrode formed in step c) into a sensor device, suitably an electrochemical sensor device. Therefore the present invention may provide a method of forming a sensor device comprising the electrode. With the benefit of the details of the present invention disclosed herein, a person skilled in the art would be able to manufacture a suitable sensor device incorporating the electrode of the first aspect, by using suitable parts, configurations and techniques from known electrochemical sensor devices. For example, the sensor device may be configured as a reusable or single use sensor device or may be integrated into a microfluidics system configured to process a sample containing the target compound or biomarker.

According to a second aspect of the present invention, there is provided an electrode for a sensor device comprising a substrate, a carbon nanofibre layer and an enzyme immobilised on the carbon nanofibre layer, wherein the carbon nanofibre layer comprises mesoporous carbon nanofibers and the enzyme is immobilised in the pores of the mesoporous carbon nanofibers, wherein the carbon nanofibre layer is formed from fibres comprising lignin and a second polymeric material, wherein the second polymeric material is immiscible with lignin.

The electrode of this second aspect may have any of the suitable features and advantages described in relation to the first aspect. Suitably the electrode of this second aspect is formed by the method of the first aspect.

Suitably the carbon nanofibre layer comprises a crosslinked network of mesoporous carbon nanofibers, as described in relation to the first aspect.

The carbon nanofibre layer is formed from fibres comprising lignin and a second polymeric material, wherein the second polymeric material is immiscible with lignin, as described in relation to the first aspect. Suitably wherein the second polymeric material is polylactic acid.

Suitably the fibres comprising lignin and the second polymeric material also comprise a crosslinking agent.

Suitably the enzyme immobilised on the carbon nanofibre layer is glucose oxidase.

According to a third aspect (not part of the invention), there is provided an electrode for a sensor device comprising a substrate, a carbon nanofibre layer and an enzyme immobilised on the carbon nanofibre layer, wherein the carbon nanofibre layer is formed from fibres comprising lignin and a second polymeric material which is immiscible with lignin, suitably polylactic acid.

The electrode of this third aspect may have any of the suitable features and advantages described in relation to the first aspect and the second aspect. Suitably the electrode of this third aspect is formed by the method of the first aspect.

According to fourth aspect of the present invention, there is provided a sensor device comprising an electrode according to the second aspect.

Suitably the sensor device is an electrochemical sensor device, for example a glucose sensor for measuring the glucose concentration of a liquid sample such as a saliva sample.

According to a fifth aspect of the present invention, there is provided a use of a mesoporous carbon nanofibre material for immobilising an enzyme on an electrode of a sensor device; wherein the mesoporous carbon nanofibre material is formed from fibres comprising lignin and a second polymeric material, wherein the second polymeric material is immiscible with lignin.

The mesoporous carbon nanofibre material may have any of the suitable features and advantages described in relation to the first aspect.

The use of this fifth aspect suitably provides an effective immobilisation of the enzyme, for example glucose oxidase, in the mesopores of the mesoporous carbon nanofibre material and provides an electrically conductive pathway between the enzyme and the electrode, in order to enable the enzyme to affect a voltage output of the electrode when a target compound or biomarker interacts with the enzyme.

Suitably the mesoporous carbon nanofibre material is formed from lignin, a second polymeric material and a crosslinking agent, suitably wherein the second polymeric material is polylactic acid. In some embodiments, the use is for immobilising glucose oxidase on an electrode of a glucose sensor device.

According to a sixth aspect of the present invention, there is provided a method of detecting a target compound or biomarker, the method comprising the steps of:
1) providing a sensor device comprising an electrode, wherein the electrode comprises a substrate, a carbon nanofibre layer and an enzyme immobilised on the carbon nanofibre layer, wherein the carbon nanofibre layer comprises mesoporous carbon nanofibers and the enzyme is immobilised in the pores of the mesoporous carbon nanofibers; wherein the mesoporous carbon nanofibre material is formed from fibres comprising lignin and a second polymeric material, wherein the second polymeric material is immiscible with lignin.
2) exposing the electrode to a sample comprising said target compound or biomarker so that the sample contacts the enzyme immobilised on the carbon nanofibre layer;
3) detecting an electrical signal produced in the electrode when said target compound or biomarker interacts with the enzyme immobilised on the carbon nanofibre layer.

The sensor device and electrode provided in step 1) of the method are suitably as described in the first, second and fourth aspects of the present invention.

Suitably the steps of the method of this sixth aspect are carried out in the order of step 1) followed by step 2) followed by step 3).

Suitably step 2) involves depositing the sample onto the carbon nanofibre layer, suitably to an exposed surface of the carbon nanofibre layer, suitably through an opening provided on the sensor device. Suitably the sample is a liquid sample, suitably a bodily fluid of a patient, for example blood or saliva. Suitably the sample is a saliva sample taken from a patient.

Suitably step 3) involves measuring a change in voltage output of the electrode on exposure of the electrode to the sample containing said target compound or biomarker and correlating said change in voltage output with a concentration of said target compound or biomarker in contact with the carbon nanofibre layer and enzyme in order to determine the concentration of said target compound or biomarker in the sample. Suitably the sensor device is calibrated using samples with known concentrations of said target compound or biomarker.

In some embodiments, the enzyme immobilised on the carbon nanofibre layer is glucose oxidase and said biomarker is glucose. Suitably the method provides a measurement of the concentration of glucose in a saliva sample of a patient, for example a diabetes patient.

### Examples

### Materials

Alcell Organosolv hardwood lignin (AL) was obtained from Tecnaro GmbH. Polylactic Acid (PLA) (Ingeo Biopolymer 3001D) injection molding grade was supplied by Natureworks, Minnetonka. Dimethylformamide (DMF), Tetrahydrofuran (THF), Methylene diphenyl diisocyanate (MDI), Potassium Ferricyanide, Potassium Chloride, Disodium Phosphate Dibasic Dihydrate, Sodium Phosphate Monobasic Monohydrate, Calcium Chloride, Nafion (NF) solution at 5%, Glucose and Glucose Oxidase (GOx) from Aspergillus Niger were obtained from Sigma Aldrich. Water used was at Pure grade. Phosphate Buffer Solution (PBS) was prepared at 0.05 mol.L⁻¹ and given pH using disodium phosphate dibasic dihydrate and sodium phosphate monobasic monohydrate dissolved in pure grade water. The solution was prepared according to the Henderson-Hasselbalch equation and the pH adjusted by addition of hydroxide sodium at 1 mol.L⁻¹ (Sigma Aldrich).

### Carbon nanofibre electrode preparation

Carbon nanofibres were formed on a gold-plated quartz electrode purchased from AdValue Technology (Tucson, AZ, USA). The quartz slides were cut to a square shaped electrode of a surface area of 1/16 inch² and gold plated for 6 min using a gold sputtering apparatus. The plates were then mounted on the collector plate of a home designed electrospinner for electrospinning.

An electrospinning solution was prepared by diluting 10% of binder polymer (PLA) in a THF and DMF solution at 50°C for 2 hours. AL was then added to the solution and stirred for 30 minutes, followed by MDI. The solid content of the solution was composed of 46.5 % AL, 50 % PLA and 3.5 % MDI. The solution was then electrospun for one hour, at an infusion rate of 30 µL.min⁻¹, with a distance needle to collector plate of 10 cm and a potential difference set at 7.7 kV.

After electrospinning, the quartz plates were dismounted and left to dry in ambient atmosphere for 24h. Prior to carbonisation, they were subjected to a thermal oxidation process to allow for crosslinking. The stabilisation was performed in a Binder FD-23 heating chamber (Tuttinger, Germany) using 1°C per min ramps with isothermals as shown in Table 1.

**Table 1: Thermal Stabilisation ramps**

| Ramp | Start Temperature | Target Temperature | Isothermal at target temperature |
|---|---|---|---|
| 1 °C.min⁻¹ | 20 °C | 150 °C | 14 hours |
| 1 °C.min⁻¹ | 150 °C | 170 °C | 2 hours |
| 1 °C.min⁻¹ | 170 °C | 200 °C | 2 hours |
| 1 °C.min⁻¹ | 200 °C | 250 °C | 2 hours |

The stabilised AL/binder fibres were finally carbonised under nitrogen flow in a tubular furnace (Lenton, UK) heating from room temperature to 900°C at 10°C.min⁻¹ and kept at 900°C for 30 minutes.

### Characterisation

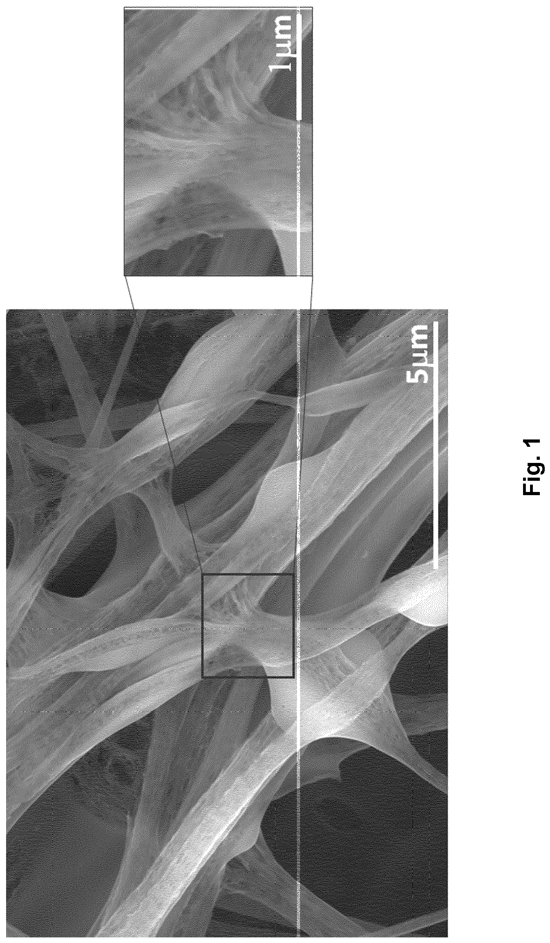

The structure and morphology of the pores were studied using SEM (Hitachi SU-70). Figure 1 shows the SEM images of the AL-PLA carbon nanofibres (CnF) after carbonisation which show the interconnected, well defined network of nanofibres. The formation of this structure is promoted by the use of crosslinker, such as MDI in this case, that avoids the fusion of the material during the stabilisation phase. In particular, the Al-PLA CnF show the cavities in the form of macropores (100 nm). These are created by the sacrificial nature of PLA, which evaporates during the carbonisation process as its carbon yield is below 3%. The size and shape of the pores was obtained using Brunauer-Emmett-Teller (BET) surface area analysis and the results are shown in Table 2.

**Table 2: Pores Volumes and Surfaces from BET results.**

| | Vₚₒᵣₑₛ (cm³.g⁻¹) | Vₘₑₛₒ (cm³.g⁻¹) | Vₘₑₛₒ/Vₚₒᵣₑₛ (%) | S_{BET} (m².g⁻¹) | Sₑₓₜₑᵣₙₐₗ (m².g⁻¹) |
|---|---|---|---|---|---|
| AL -PLA | 0.690 | 0.306 | 50.2 | 670 | 50.6 |

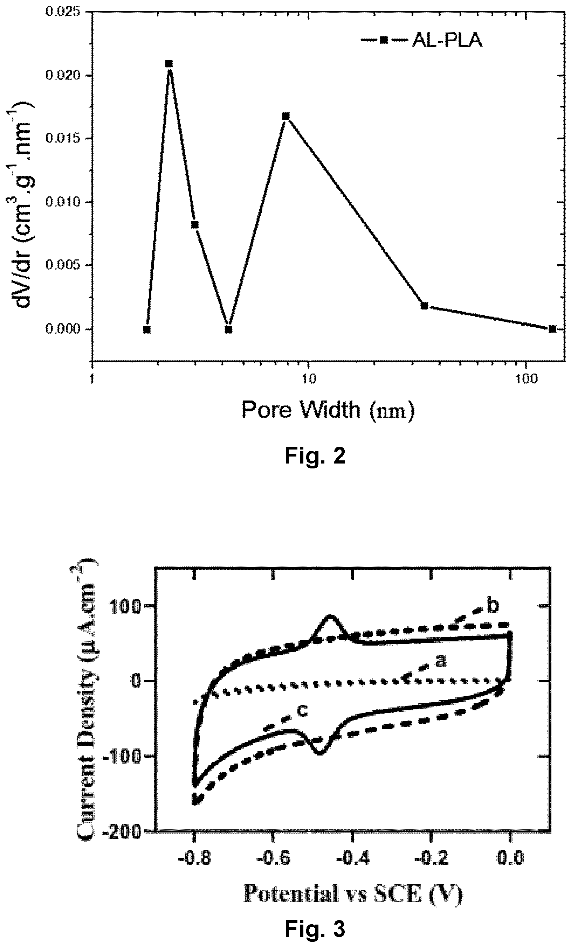

Figure 2 shows the pore width distribution for the AL-PLA CnF. This pore width distribution shows two populations of pores, at approximately 2.2 nm and above 10 nm. Noticeably, the AL-PLA CnF presents a higher number of small mesopores, which population was found to be advantageous for the immobilisation of enzymes such as GOx.

The pore size distribution was found to be advantageous for the immobilisation and successful electron transfer between GOx and the sensing platform with pores between 2-4 nm offering the best enzyme activity. Pore volumes of the CnF material appear to be influenced by the choice of binder polymer. The AL-PLA polymer blend provides a relative mesopores volume ratio higher than 50%. Also, the external surface, where the glucose oxidase is reportedly confined, is increased for the AL-PLA blend compared to a polymer blend comprising lignin and a polymer which is miscible with lignin, such as thermoplastic elastomer polyurethane (TPU). These results show that the CnF derived from the AL-PLA blend have a mesoporous structure to allow for the confinement of GOx enzyme. The mesopores are believed to be contained within the cavities created by the sacrifice of PLA, offering a unique structure for the enzyme to be retained within.

### Fabrication of the Gold/CnF/GOx/NF biosensor

The quartz plate was mounted as a working electrode using a glass slide as support. Electric contacts were made using silver conductive paste (Radiospare, UK) and protected with quick set epoxy patch (Radiospare, UK). A surface area of 1 cm² was left free for sensing. The surface area A of the electrode was checked by cyclic voltammetry using a potentiostat (Parstat 2273, Princeton Applied Research, Ametek, Inc, USA) on a 3 electrodes set-up (saturated calomel reference electrode, Platinum counter electrode and prepared working electrode) in a ferricyanide solution of concentration C = 5 mM in 0.1 M KCI. The scan rates were varied between 10 to 200 mV.s⁻¹ and the slope of the peak anodic intensity Iₚ as a function of the square root of the scan rate was used in the Randles-Sevcik equation.

The enzyme immobilisation was achieved by drop casting 300 µL of GOx in PBS at 5 mg.mL⁻¹. The electrode was left to dry for 4 hours in a heat chamber at 37°C before adding 50 µL of Nafion on the surface. The mounted electrode was again left to dry for 24 h before a water rinse.

### Electrochemical measurements

For cyclic voltammetry (CV) measurements a potentiostat (Parstat 2273, Princeton Applied Research, Ametek Inc, USA) was used. The electrochemical measurements were performed using three electrodes, a saturated calomel reference electrode (SCE), a platinum counter electrode and the prepared working electrode. For all electrochemical measurements, PBS was prepared at 0.05 mol.L⁻¹ and pH 7 was used as the electrolyte. Prior to measurements, the PBS was degassed by heating under stirring to 60°C before cooling down under either N₂ or air bubbling to saturate the solution.

Figure 3 shows CycloVoltametry plots of different modified gold electrodes in 0.05 mol.L⁻¹ N₂ saturated PBS at 50 mV.s⁻¹ as (a) GOx, (b) AL-PLA CnF, (c) AL-PLA CnF/GOx. The gold electrode acts as a signal collector, allowing for the electrons to propagate through the carbon layer to the enzymes.

The GOx/Gold electrode does not produce any redox current (curve a), as the redox centres in GO_{X} are not activated by the bare surface. The AL-PLA CnF electrode does not produce any redox peaks either when no GOx is immobilised. The increased surface area of this material in the electrode is displayed by the square CV curve typical of a double layer capacitance (curve b). When GOx is immobilised on its surface, the AL-PLA CnF exhibits strong redox activity with a cathodic peak at - 0.48 V and an anodic peak at -0.45 V vs SCE (curve c). This redox activity can be attributed to the DET on FAD/FADH₂ redox centres for GOx, as their redox potential is consistent with that reported at pH = 7.0.

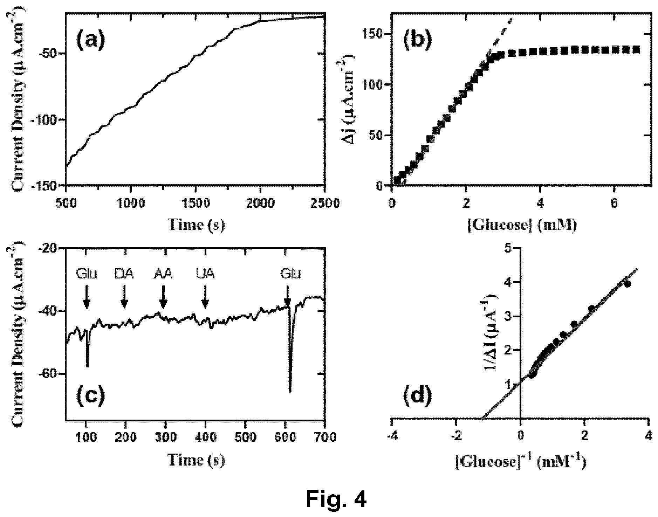

Chronoamperometry experiments were performed using the same 3 electrodes system as described above. The potential of the solution was kept at -0.56 V. 100 mL of a concentrated solution of glucose in PBS was added every 100 seconds to a PBS solution at 0.05 mol.L⁻¹ at pH 7 and 25°C. The calibration curve was corrected for dilution. Figure 4 shows the determination of the biosensor performance performed in O₂ saturated PBS at pH = 7 and 0.05 mol.L⁻¹. (a) Chronoamperometry performed at E = -0.56 V with 150 mM of glucose added every 100s. (b) Calibration curve of the sensor determined from the points of (a) in the steady state current response. (c) Effect of interfering species at 0.5 mM (Dopamine DA, Ascorbic Acid AA and Uric Acid UA). (d) Lineweaver-Burk plot based on the reciprocal of the calibration curve, for the determination of Kₘ the Michaelis-Menten constant.

The performance of the sensor was determined using chronoamperometry i-t. Figure 4 a) shows the current-time response of the electrode to successive injections of glucose in a PBS solution at 0.05 M and pH = 7 at -0.56 V. The decrease of the steady state current (see inset) is plotted against the glucose concentration. The sensor presents a linear response to the adding of glucose between 0.15-2.7 mM, with a sensitivity of 50 µA.mM⁻¹.cm⁻² as seen on its calibration curve (Figure 4 b)). The limit of detection (LoD) was calculated as 89 µmol.L⁻¹ for a signal to noise ratio of 3. The sensor is therefore capable of detecting glucose at 16 ppm.

The saturation reached by GOx at high concentration of glucose is typical of substrate-enzyme kinetics as described by the Michaelis-Menten constant (Kₘ). Kₘ was derived from the Lineweaver-Burk equation. The Kₘ was calculated as 600 mM. This low value in comparison with reported GOx-based biosensors shows that the glucose has a high affinity towards the surface of the electrode.

Finally, the sensor shows good selectivity to glucose as displayed in Figure 4 c), where the current response to the addition of DA, AA and UA stays below the noise level.

In summary, the present invention provides an electrode for an electrochemical sensor device. The electrode comprises a substrate, a carbon nanofibre layer and an enzyme immobilised on the carbon nanofibre layer. The carbon nanofibre layer comprises mesoporous carbon nanofibers and the enzyme is immobilised in the pores of the mesoporous carbon nanofibers. The carbon nanofibre layer is formed from lignin and a polymeric material which is immiscible in lignin, such as polylactic acid, through a process of stabilisation and carbonisation which provides a conductive carbon nanofibre framework comprising mesopores suitable for immobilisation of the enzyme. The enzyme immobilised in the carbon nanofibre layer can function by interacting with a target compound or biomarker in a sample solution applied to the electrode which produces a measurable electrochemical change in the electrode. A method of forming the electrode, a sensor device comprising the electrode, a use of a mesoporous carbon nanofibre material for immobilising an enzyme in a sensor device and a method of detecting a target compound or biomarker using the electrode are also provided. The results discussed above demonstrate that the immobilisation of enzymes in mesoporous carbon nanofibres produced from polylactic acid and lignin used in the present invention can provide an effective electrode for a sensor device and therefore such electrodes can be advantageously prepared from these low-cost sustainable materials.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1% by weight of non-specified components.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

For the avoidance of doubt, wherein amounts of components in a composition are described in wt%, this means the weight percentage of the specified component in relation to the whole composition referred to. For example, "the fibres comprise at least 30 wt% lignin" means that 30 wt% of the fibres is provided by lignin.

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

## Claims

1. A method of forming an electrode for a sensor device, the method comprising the steps of:
a) forming fibres on a substrate, the fibres comprising lignin and a second polymeric material, wherein the second polymeric material is immiscible with lignin;
b) carbonising the fibres formed in step a) to provide a carbon nanofibre layer on the substrate, wherein the carbon nanofibre layer comprises mesoporous carbon nanofibers; and
c) immobilising an enzyme on the carbon nanofibre layer, in mesopores of the carbon nanofibres.

2. The method according to claim 1, wherein the fibres of step a) comprise a crosslinking agent.

3. The method according to claim 1 or claim 2, wherein the fibres of step a) comprise 30-70 wt% lignin and 30-70 wt% of the second polymeric material.

4. The method according to any preceding claim wherein the second polymeric material is polylactic acid.

5. The method according to any preceding claim, wherein the substrate comprises a conductive layer, preferably comprising gold.

6. The method according to any preceding claim, wherein the method comprises a step a2) of stabilising the fibres by exposing the fibres on the substrate to a temperature of from 100°C to 300°C;
preferably wherein step b) of carbonising the fibres involves exposing the fibres on the substrate to a temperature of at least 700°C.

7. The method according to any preceding claim, wherein the carbon nanofibers produced in step b) are porous or mesoporous with a BET surface area of at least 500 m²g⁻¹.

8. The method according to any preceding claim, wherein the enzyme of step c) is glucose oxidase and the sensor device is a glucose sensor.

9. An electrode for a sensor device comprising a substrate, a carbon nanofibre layer and an enzyme immobilised on the carbon nanofibre layer, wherein the carbon nanofibre layer comprises mesoporous carbon nanofibers and the enzyme is immobilised in the pores of the mesoporous carbon nanofibers wherein the carbon nanofibre layer is formed from fibres comprising lignin and a second polymeric material, wherein the second polymeric material is immiscible with lignin.

10. The electrode of claim 9, wherein the fibres comprising lignin and the second polymeric material comprise a crosslinking agent.

11. The electrode of claims 9 or 10, wherein the enzyme is glucose oxidase.

12. A sensor device comprising an electrode according to any of claims 9 to 11.

13. Use of a mesoporous carbon nanofibre material for immobilising an enzyme in a sensor device; wherein the mesoporous carbon nanofibre material is formed from fibres comprising lignin and a second polymeric material, wherein the second polymeric material is immiscible with lignin.

14. The use of claim 13 wherein the mesoporous carbon nanofibre material is formed from polylactic acid, lignin and a crosslinking agent.

15. A method of detecting a target compound or biomarker, the method comprising the steps of:
1) providing a sensor device comprising an electrode, wherein the electrode comprises a substrate, a carbon nanofibre layer and an enzyme immobilised on the carbon nanofibre layer, wherein the carbon nanofibre layer comprises mesoporous carbon nanofibers and the enzyme is immobilised in the pores of the mesoporous carbon nanofibers; wherein the mesoporous carbon nanofibre is formed from fibres comprising lignin and a second polymeric material, wherein the second polymeric material is immiscible with lignin;
2) exposing the electrode to a sample comprising said target compound or biomarker so that the sample contacts the enzyme immobilised on the carbon nanofibre layer;
3) detecting an electrical signal produced in the electrode when said target compound or biomarker interacts with the enzyme immobilised on the carbon nanofibre layer; preferably wherein the enzyme immobilised on the carbon nanofibre layer is glucose oxidase and said biomarker is glucose.

## Patentansprüche

1. Verfahren zur Bildung einer Elektrode für eine Sensorvorrichtung, wobei das Verfahren die folgenden Schritte umfasst:
a) das Bilden von Fasern auf einem Substrat, wobei die Fasern Lignin und ein zweites polymeres Material umfassen, wobei das zweite polymere Material mit Lignin nicht mischbar ist;
b) das Carbonisieren der in Schritt a) gebildeten Fasern, wodurch auf dem Substrat eine Kohlenstoff-Nanofaserschicht bereitgestellt wird, wobei die Kohlenstoff-Nanofaserschicht mesoporöse Kohlenstoff-Nanofasern umfasst, und
c) das Immobilisieren eines Enzyms auf der Kohlenstoff-Nanofaserschicht in Mesoporen der Kohlenstoff-Nanofasern.

2. Verfahren nach Anspruch 1, wobei die Fasern von Schritt a) ein Vernetzungsmittel umfassen.

3. Verfahren nach Anspruch 1 oder Anspruch 2, wobei die Fasern von Schritt a) 30 - 70 Gew.-% Lignin und 30 - 70 Gew.-% des zweiten polymeren Materials umfassen.

4. Verfahren nach einem der vorhergehenden Ansprüche, wobei das zweite polymere Material Polymilchsäure ist.

5. Verfahren nach einem der vorhergehenden Ansprüche, wobei das Substrat eine leitfähige Schicht umfasst, die vorzugsweise Gold umfasst.

6. Verfahren nach einem der vorhergehenden Ansprüche, wobei das Verfahren einen Schritt a2) eines Stabilisierens der Fasern durch ein Exponieren der Fasern auf dem Substrat gegenüber einer Temperatur von 100 °C bis 300 °C umfasst;
wobei Schritt b) des Carbonisierens der Fasern vorzugsweise ein Exponieren der Fasern auf dem Substrat gegenüber einer Temperatur von mindestens 700 °C einschließt.

7. Verfahren nach einem der vorhergehenden Ansprüche, wobei die in Schritt b) erzeugten Kohlenstoff-Nanofasern porös oder mesoporös mit einer spezifischen BET-Oberfläche von mindestens 500 m²g⁻¹ sind.

8. Verfahren nach einem der vorhergehenden Ansprüche, wobei es sich bei dem Enzym von Schritt c) um Glucoseoxidase handelt und bei der Sensorvorrichtung um einen Glucosesensor handelt.

9. Elektrode für eine Sensorvorrichtung, die ein Substrat, eine Kohlenstoff-Nanofaserschicht und ein auf der Kohlenstoff-Nanofaserschicht immobilisiertes Enzym umfasst, wobei die Kohlenstoff-Nanofaserschicht mesoporöse Kohlenstoff-Nanofasern umfasst und das Enzym in den Poren der mesoporösen Kohlenstoff-Nanofasern immobilisiert ist, wobei die Kohlenstoff-Nanofaserschicht aus Fasern gebildet ist, die Lignin und ein zweites polymeres Material umfassen, wobei das zweite polymere Material mit Lignin nicht mischbar ist.

10. Elektrode nach Anspruch 9, wobei die Fasern, die Lignin und das zweite polymere Material umfassen, ein Vernetzungsmittel umfassen.

11. Elektrode nach den Ansprüchen 9 oder 10, wobei es sich bei dem Enzym um Glucoseoxidase handelt.

12. Sensorvorrichtung, die eine Elektrode nach einem der Ansprüche 9 bis 11 umfasst.

13. Verwendung eines mesoporösen Kohlenstoff-Nanofasermaterials zur Immobilisierung eines Enzyms in einer Sensorvorrichtung; wobei das mesoporöse Kohlenstoff-Nanofasermaterial aus Fasern gebildet ist, die Lignin und ein zweites polymeres Material umfassen, wobei das zweite polymere Material mit Lignin nicht mischbar ist.

14. Verwendung nach Anspruch 13, wobei das mesoporöse Kohlenstoff-Nanofasermaterial aus Polymilchsäure, Lignin und einem Vernetzungsmittel gebildet ist.

15. Verfahren zum Nachweis einer Zielverbindung oder eines Biomarkers, wobei das Verfahren die folgenden Schritte umfasst:
1) das Bereitstellen einer Sensorvorrichtung, die eine Elektrode umfasst, wobei die Elektrode ein Substrat, eine Kohlenstoff-Nanofaserschicht und ein auf der Kohlenstoff-Nanofaserschicht immobilisiertes Enzym umfasst, wobei die Kohlenstoff-Nanofaserschicht mesoporöse Kohlenstoff-Nanofasern umfasst und das Enzym in den Poren der mesoporösen Kohlenstoff-Nanofasern immobilisiert ist, wobei die mesoporöse Kohlenstoff-Nanofaser aus Fasern gebildet ist, die Lignin und ein zweites polymeres Material umfassen, wobei das zweite polymere Material mit Lignin nicht mischbar ist;
2) das Exponieren der Elektrode gegenüber einer Probe, welche die Zielverbindung oder einen Biomarker umfasst, sodass die Probe mit dem auf der Kohlenstoff-Nanofaserschicht immobilisierten Enzym in Kontakt gerät;
3) das Erfassen eines elektrischen Signals, das in der Elektrode erzeugt wird, wenn die Zielverbindung oder der Biomarker mit dem auf der Kohlenstoff-Nanofaserschicht immobilisierten Enzym in Wechselwirkung tritt;
wobei es sich bei dem auf der Kohlenstoff-Nanofaserschicht immobilisierten Enzym vorzugsweise um Glucoseoxidase handelt und bei dem Biomarker um Glucose handelt.

## Revendications

1. Procédé de formation d'une électrode pour un dispositif capteur, le procédé comprenant les étapes de :
a) formation de fibres sur un substrat, les fibres comprenant de la lignine et un second matériau de polymère, le second matériau de polymère étant non miscible avec la lignine ;
b) carbonisation des fibres formées à l'étape a) pour fournir une couche de nanofibres de carbone sur le substrat, dans lequel la couche de nanofibres de carbone comprend des nanofibres de carbone mésoporeuses ; et
c) immobilisation d'une enzyme sur la couche de nanofibres de carbone, dans des mésopores des nanofibres de carbone.

2. Procédé selon la revendication 1, dans lequel les fibres de l'étape a) comprennent un agent de réticulation.

3. Procédé selon la revendication 1 ou la revendication 2, dans lequel les fibres de l'étape a) comprennent 30 à 70 % en poids de lignine et 30 à 70 % en poids du second matériau de polymère.

4. Procédé selon l'une quelconque des revendications précédentes, dans lequel le second matériau de polymère est un poly(acide lactique).

5. Procédé selon l'une quelconque des revendications précédentes, dans lequel le substrat comprend une couche conductrice, comprenant de préférence de l'or.

6. Procédé selon l'une quelconque des revendications précédentes, dans lequel le procédé comprend une étape a2) de stabilisation des fibres en exposant les fibres sur le substrat jusqu'à une température de 100 °C à 300 °C ;
de préférence, dans lequel l'étape b) de carbonisation des fibres implique une exposition des fibres sur le substrat à une température d'au moins 700 °C.

7. Procédé selon l'une quelconque des revendications précédentes, dans lequel les nanofibres de carbone produites dans l'étape b) sont poreuses ou mésoporeuses avec une superficie BET d'au moins 500 m²g⁻¹.

8. Procédé selon l'une quelconque des revendications précédentes, dans lequel l'enzyme de l'étape c) est une glucose oxydase et le dispositif capteur est un capteur de glucose.

9. Électrode pour dispositif capteur comprenant un substrat, une couche de nanofibres de carbone et une enzyme immobilisée sur la couche de nanofibres de carbone, dans laquelle la couche de nanofibres de carbone comprend des nanofibres de carbone mésoporeuses et l'enzyme est immobilisée dans les pores des nanofibres de carbone mésoporeuses, la couche de nanofibres de carbone étant formée à partir de fibres comprenant de la lignine et un second matériau de polymère, le second matériau de polymère étant non miscible avec la lignine.

10. Électrode selon la revendication 9, dans laquelle les fibres comprenant de la lignine et le second matériau de polymère comprennent un agent de réticulation.

11. Électrode selon la revendication 9 ou 10, dans laquelle l'enzyme est une glucose oxydase.

12. Dispositif capteur comprenant une électrode selon l'une quelconque des revendications 9 à 11.

13. Utilisation d'un matériau de nanofibres de carbone mésoporeuses pour immobiliser une enzyme dans un dispositif capteur ; dans laquelle le matériau de nanofibres de carbone mésoporeuses est formé à partir de fibres comprenant de la lignine et un second matériau de polymère, dans laquelle le second matériau de polymère est non miscible avec la lignine.

14. Utilisation selon la revendication 13, dans laquelle le matériau de nanofibres de carbone mésoporeuses est formé à partir de poly(acide lactique), de lignine et d'un agent de réticulation.

15. Procédé de détection d'un composé ou biomarqueur cible, le procédé comprenant les étapes de :
1) fourniture d'un dispositif capteur comprenant une électrode, dans lequel l'électrode comprend un substrat, une couche de nanofibres de carbone et une enzyme immobilisée sur la couche de nanofibres de carbone, dans lequel la couche de nanofibres de carbone comprend des nanofibres de carbone mésoporeuses et l'enzyme est immobilisée dans les pores des nanofibres de carbone mésoporeuses ; dans lequel la nanofibre de carbone mésoporeuse est formée à partir de fibres comprenant de la lignine et un second matériau de polymère, dans lequel le second matériau de polymère est non miscible avec la lignine ;
2) exposition de l'électrode à un échantillon comprenant ledit composé ou biomarqueur cible de sorte que l'échantillon entre en contact avec l'enzyme immobilisée sur la couche de nanofibres de carbone ;
3) détection d'un signal électrique produit dans l'électrode lorsque ledit composé ou biomarqueur cible interagit avec l'enzyme immobilisée sur la couche de nanofibres de carbone ;
de préférence, dans lequel l'enzyme immobilisée sur la couche de nanofibres de carbone est une glucose oxydase et ledit biomarqueur est le glucose.
